# EUROPÄISCHE PATENTSCHRIFT

(11) **EP 1 558 384 B1**
(45) Veröffentlichungstag und Bekanntmachung des Hinweises auf die Patenterteilung: **25.03.2015**
(21) Anmeldenummer: 03750375.2
(22) Anmeldetag: 26.09.2003
(51) Int. Cl.: B01J 31/18, B01J 23/46, C07F 9/50, C07F 15/00, C07F 19/00, C07F 9/6571, B01J 31/24, C07B 53/00, C07C 67/303, C07F 9/46, C07F 9/52, C07F 9/6568, C07F 9/6574, C07F 9/6584, C07C 231/12

(54) **MISCHUNGEN VON CHIRALEN MONOPHOSPHOR-VERBINDUNGEN ALS LIGANDEN-SYSTEME FÜR DIE ASYMMETRICHE ÜBERGANGSMETALL-KATALYSE**
MIXTURES OF CHIRAL MONOPHOSPHORUS COMPOUNDS USED AS LIGAND SYSTEMS FOR ASYMMETRIC TRANSITION METAL CATALYSIS
MELANGES DE COMPOSES CHIRAUX A UN ATOME DE PHOSPHORE EN TANT QUE SYSTEMES DE LIGANDS POUR LA CATALYSE ASYMETRIQUE A L'AIDE DE METAUX DE TRANSITION

(30) Priorität: 11.10.2002 DE 10247633
(43) Veröffentlichungstag der Anmeldung: 03.08.2005
(73) Patentinhaber: Studiengesellschaft Kohle mbH, 45470 Mülheim an der Ruhr (DE)
(72) Erfinder: REETZ, Manfred, T., 45470 Mülheim an der Ruhr (DE); SELL, Thorsten, 60487 Frankfurt am Main (DE); MEISWINKEL, Andreas, 45470 Mülheim an der Ruhr (DE); MEHLER, Gerlinde, 45470 Mülheim an der Ruhr (DE)
(86) Internationale Anmeldenummer: PCT/DE2003/003226
(87) Internationale Veröffentlichungsnummer: WO 2004/035208

(56) Entgegenhaltungen:
- WO-A-01/94278
- US-A- 5 360 938
- CHEN W ET AL: "Asymmetric activation of conformationally flexible monodentate phosphites for enantioselective hydrogenation" TETRAHEDRON LETTERS, ELSEVIER SCIENCE PUBLISHERS, AMSTERDAM, NL, Bd. 42, Nr. 49, 3. Dezember 2001 (2001-12-03), Seiten 8737-8740, XP004321537 ISSN: 0040-4039
- DATABASE REGISTRY [Online] CA; Registry number: RN 174367-22-7, 20. März 1996 (1996-03-20) XP002271188
- REETZ M T ET AL: "Mixtures of chiral and achiral monodentate ligands in asymmetric Rh-catalyzed olefin hydrogenation: reversal of enantioselectivity" TETRAHEDRON LETTERS, ELSEVIER SCIENCE PUBLISHERS, AMSTERDAM, NL, Bd. 44, Nr. 24, 9. Juni 2003 (2003-06-09), Seiten 4593-4596, XP004425274 ISSN: 0040-4039
- REETZ, M. ET AL: "A new principle in combinatorial asymmetric transition-metal catalysis: mixtures of chiral monodentate P ligands" ANGEW. CHEM. INT. ED., Bd. 42, Nr. 7, 2003, Seiten 790-793, XP002270700
- PENA, D. ET AL: "Improving conversion and enantioselectivity in hydrogenation by combining different monodentate phosphoramidites; a new combinatorial approach in asymmetric catalysis" ORG. BIOMOL. CHEM., Bd. 1, 2003, Seiten 1087-1089, XP002270701
- BERG VAN DEN M ET AL: "Highly enantioselective Rhodium catalyzed hydrogenation with monodentate ligands" JOURNAL OF THE AMERICAN CHEMICAL SOCIETY, AMERICAN CHEMICAL SOCIETY, WASHINGTON, DC, US, Bd. 122, Nr. 46, 22. November 2000 (2000-11-22), Seiten 11539-11540, XP002249650 ISSN: 0002-7863 in der Anmeldung erwähnt

## Beschreibung

Die vorliegende Erfindung beinhaltet den überraschenden Befund, dass Mischungen von zwei oder mehreren chiralen Monophosphor-Verbindungen (also Verbindungen mit einem P-Atom) oder Mischungen, bestehend aus mindestens einer chiralen und mindestens einer achiralen Monophosphor-Verbindung, ausgezeichnete Ligandensysteme in der asymmetrischen Übergangsmetall-Katalyse darstellen. Es handelt sich um grundsätzlich neue Verfahren auf dem Gebiet der enantioselektiven Übergangsmetall-Katalyse, bei denen bekannte oder neue chirale Mono-P-Verbindungen eingesetzt werden. Ferner handelt es sich um einen strukturell neuen Typ von chiralen Übergangsmetell-Katalysatoren, denn am Metall sind zwei (oder mehrere) unterschiedliche Monophosphor-Verbindungen, von denen mindestens eine eine chirale Monophosphor-Verbindung ist, gebunden. Solche Metall-Komplexe wurden in der Literatur noch nie erwähnt.

Enantioselektive Übergangsmetall-katalysierte Prozesse haben in den letzten 20 Jahren industriell an Bedeutung gewonnen, so z. B. die Übergangsmetall-katalysierte asymmetrische Hydrierung (B. Cornils, W. A. Herrmann, Applied Homogeneous Catalysis with Organometallic Compounds, Wiley-VCH, Weinheim (1996); W. S. Knowles, Angew. Chem., 114, 2096 (2002); R. Noyori, Angew. Chem., 114, 2108 (2002)). Die dazu erforderlichen Liganden sind häufig chirale phosphorhaltige Liganden (P-Liganden), z. B. Phosphane, Phosphonite, Phosphinite, Phosphite oder Phosphoramidite, die an den Übergangsmetallen gebunden sind. Als typische Beispiele seien Rhodium-, Ruthenium- oder Iridium-Komplexe von optisch aktiven Diphosphanen wie BINAP (A. Miyashita, A. Yasuda, H. Takaya, K. Toriumi, T. Ito, T. Souchi, R. Noyori, J. Am. Chem. Soc., 102, 7932 (1980)), DuPHOS (M. J. Burk, M. F. Gross, J. P. Martinez, J. Am. Chem. Soc., 117, 9375 (1995)), BICP (G. Zhu, P. Cao, Q. Jiang, X. Zhang, J. Am. Chem. Soc., 119, 1799 (1997) und BPE (M. J. Burk, Y. M. Wang, J. R. Lee, J. Am. Chem. Soc., 118, 5142 (1996)) erwähnt. Die Entwicklung chiraler Liganden erfordert ein kostspieliges Verfahren, bestehend aus "Design" und "trial and error" (W. S. Knowles, Angew. Chem., 114, 2096 (2002)). Eine ergänzende Suchmethode ist die sogenannte kombinatorische asymmetrische Katalyse, bei der Bibliotheken von modular aufgebauten chiralen Liganden bzw. Katalysatoren hergestellt und getestet werden, wodurch die Wahrscheinlichkeit des Auffindens eines Treffers erhöht wird (M. T. Reetz, Angew. Chem., 113, 292 (2001); S. Dahmen, S. Bräse, Synthesis, 1431 (2001)). Nachteilig bei all diesen Systemen ist der relativ hohe präparative Aufwand bei der Darstellung großer Zahlen von Liganden sowie die oftmals unzureichende Enantioselektivität, die bei der Katalyse beobachtet wird. Es ist daher nach wie vor das Ziel der industriellen und akademischen Forschung, neue, billige und besonders leistungsfähige Liganden auf möglichst einfachem Weg herzustellen.

Während die meisten chiralen phosphorhaltigen Liganden chelatisierende Diphosphor-Verbindungen wie Diphosphane (W. S. Knowles, Angew. Chem., 114, 2096 (2002); R. Noyori, Angew. Chem., 114, 2108 (2002)), Diphosphite (z. B. M. T. Reetz, T. Neugebauer, Angew. Chem., 111, 134 (1999)), Diphosphinite (z. B. R. Selke. J. Organomet. Chem., 370, 249 (1989)) oder Diphosphonite (z. B. M. T. Reetz, A. Gosberg, R. Goddard, S.-M. Kyung, Chem. Commun. (Cambridge), 2077 (1998)) darstellen, die das jeweilige Übergangsmetall als Chelat-Komplex binden, stabilisieren und dabei das Ausmaß der asymmetrischen Induktion bei der Katalyse bestimmen, ist vor einiger Zeit bekannt geworden, dass bestimmte chirale Monophosphonite (z. B. M. T. Reetz, T. Sell, Tetrahedron Lett., 41, 6333 (2000); C. Claver, E. Fernandez, A. Gillon, K. Heslop, D. J. Hyett, A. Martorell, A. G. Orpen, P. G. Pringle, Chem. Commun. (Cambridge), 961 (2000)), Monophosphite (M. T. Reetz, G. Mehler, Angew. Chem., 112, 4047 (2000)) sowie Monophosphoramidite (z. B. M. van den Berg, A. J. Minnaard. E. P. Schudde, J. van Esch, A. H. M. de Vries, J. G. de Vries, B. L. Feringa, J. Am. Chem. Soc., 122, 11539 (2000)) ebenfalls effiziente Liganden sein können, so z. B. bei der Rhodiumkatalysierten asymmetrischen Hydrierung von prochiralen Olefinen. Bekannte Beispiele sind BINOL-abgeleitete Vertreter wie z. B. die Liganden I, II und III. Spektroskopische und mechanistische Studlen deuten darauf hin, dass in der Katalyse jeweils zwei Mono-P-Liganden am Metall gebunden sind. Deswegen beträgt das Metall-Ligand-Verhältnis in der Regel 1 : 2. Auch manche chirale Monophosphane des Typs R¹R²R³P können bei der Übergangsmetall-Katalyse gute Liganden sein, obgleich sie in der Regel teuer sind (z. B. W. S. Knowles, Angew. Chem., 114, 2096 (2002)).

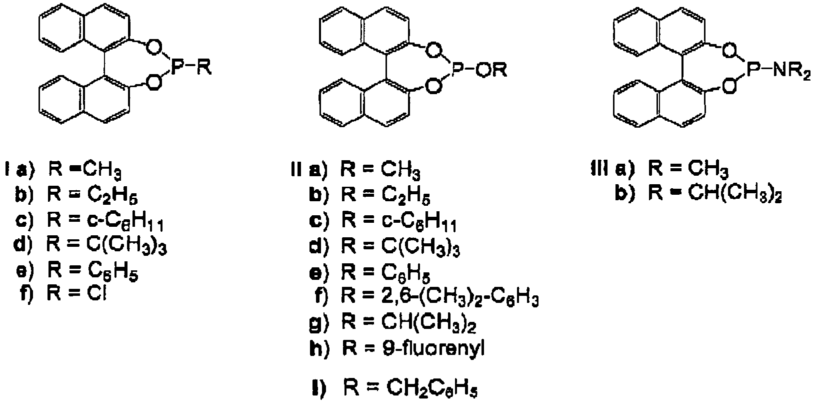

Monophosphor-haltige Liganden des Typs I - III sind besonders leicht zugänglich und können aufgrund des modularen Aufbaus sehr leicht variiert werden (I. V. Komarov, A. Bömer, Angew. Chem., 113, 1237 (2001)). Durch Variation des Restes R in I, II oder III lässt sich eine Vielzahl von chiralen Liganden aufbauen, wodurch eine Ligandenoptimierung bei einer gegebenen Übergangsmetall-katalysierten Reaktion (z. B. Hydrierung eines prochiralen Olefins. Ketons oder Imins oder Hydroformyllerung eines prochiralen Olefins) möglich ist. Leider existieren auch hier Grenzen der Methode, d. h. viele Substrate werden mit einer mäßigen oder schlechten Enantioselektivität umgesetzt, z. B. bei Hydrierungen oder Hydroformylierungen. Deshalb besteht nach wie vor der Bedarf an billigen und effektiven chiralen Liganden für die industrielle Anwendung in der Übergangsmetall-Katalyse.

Ein zentraler Bestandteil der vorliegenden Erfindung ist der überraschende Befund, dass Mischungen von zwei oder mehreren Monophosphor-Verbindungen, von denen mindestens eine chiral ist, zu höheren Enantioselektivitäten bei Übergangsmetall-katalysierten Stoffumwandlungen führen als bei der bislang üblichen Vorgehensweise unter Verwendung eines einzigen strukturell definierten Mono-P-Liganden. Diese von uns eingesetzten Übergangsmetall-Katalysatoren, bei denen mindestens zwei unterschiedliche Mono-Phosphor-Liganden (also Verbindungen mit einem P-Atom) am Metall gebunden sind, wobei mindestens ein Mono-Phosphor-Ligand chiral ist, sind neu. Diese Katalysatoren lassen sich in einer großen Zahl von unterschiedlichen Reaktionstypen zur Herstellung von chiralen organischen Verbindungen aus prochiralen organischen Verbindungen nutzen. Die optisch angereicherten oder reinen organischen Verbindungen sind bekannterweise begehrte Produkte oder Zwischenverbindungen in der Industrie, z.B. bei der Herstellung von Pharmazeutika, Pflanzenschutzmitteln und Duftstoffen.

Theoretisch kann die Methode immer dann greifen, wenn im Übergangszustand der Reaktion mindestens zwei Monophosphor-Liganden (L) am Metall (M) des aktiven Katalysators MLₓ gebunden sind. Solche Koordinationsverhältnisse sind für Metalle der Gruppen IIIb, IVb, Vb, VIb, Vllb, VIIIb, Ib und IIb sowie für die Lanthaniden und Actiniden bekannt. So kommen z. B. bei einer Mischung zwei solcher Liganden L^{a} und L^{b} drei unterschiedliche im Gleichgewicht vorliegende Katalysatoren in Frage, nämlich die traditionellen Homokombinationen ML^{a}L^{a} und ML^{b}L^{b} sowie die neuartige Heterokombination ML^{a}L^{b} (Mischkatalysator). In der Literatur findet man viele Beispiele für Homokombinationen, in jüngster Zeit z. B. die aus BINOL modular aufgebauten Monophosphonite I, Monophosphite II und das Monophosphoramidit III, die bei der Rh-katalysierten Olefinhydrierung häufig (jedoch nicht immer) hohe Enantioselektivitäten ermöglichen. Dagegen wurden Heterokombinationen ML^{a}L^{b} als Katalysatoren noch nicht beschrieben. Da In der Regel mit raschem Ligandenaustausch zu rechnen ist, dürfte ML^{a}L^{b} in Lösung kaum in reiner Form zu erzeugen sein. Das Gemisch der drei Katalysatoren kann jedoch immer dann zu einer höheren Enantioselektivität führen, wenn ML^{a}L^{b} rascher und enantioselektiver wirkt als die In reiner Form eingesetzten Katalysatoren ML^{a}L^{a} bzw. ML^{b}L^{b}, wobei die relative Menge der Liganden L^{a} und L^{b} ebenfalls eine Rolle spielen könnte.

Die vorliegende Erfindung ist daher gerichtet auf einen chiralen Übergangsmetall-Katalysator, bei dem am Metall, ausgewählt aus Rh, Ir, Ru, Nl, Pd oder Pt, mindestens zwei strukturell unterschiedliche Mono-Phosphor-Liganden gebunden sind, wobei mindestens ein Mono-Phosphor-Ligand chiral ist, der ausgewählt ist aus Mono-P-Verbindungen vom Typ B, C oder D, 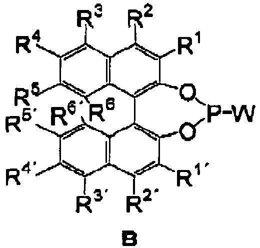 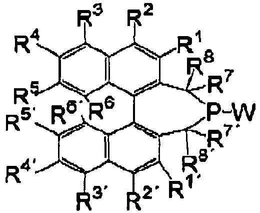 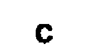 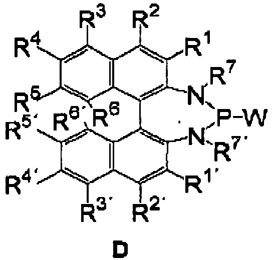 wobei W für Kohlenstoff (C), Stickstoff (N), Sauerstoff (O), Schwefel (S) oder Halogen (F, Cl, Br, I) steht und an W entsprechend seiner Anzahl von freien Valenzen weitere Atome oder Gruppen von Atomen gebunden sind,
und wobei die Reste R¹, R², R³, R⁴, R⁵, R⁶, R⁷, R⁸, R^{1'}, R^{2'}, R^{3'}, R^{4'}, R^{5'}, R^{6'}, R^{7'} und R^{8'} unabhängig voneinander aus der Reihe Wasserstoff, Halogen, gesättigte und ungesättigte lineare und verzweigte C₁-C₅₀ Alkyl-, C₆-C₅₀ Aryl-, C₁-C₅₀ Heteroaryl-, Alkinyl-, Silyl-, Nitro-, Nitril-, Ester-, Carboxyl-, Carbonyl-, Amid-, Amin-, Hydroxy-, Alkoxy-, Sulfid- und Selenidgruppen ausgewählt sind,
wobei R¹, R², R³, R⁴, R⁵, R⁶, R⁷, R⁸, R^{1'}, R^{2'}, R^{3'}, R^{4'}, R^{5'}, R^{6'}, R^{7'} und R^{8'} ihrerseits weitere Substituenten tragen oder funktionalisiert sein können.

Um dieses neue Prinzip auf dem Gebiet der asymmetrischen Übergangsmetall-Katalyse zu illustrieren, sel die enantioselektive Rh-katalysierte Olefinhydrierung eines gegebenen Substrats mit einem aus I abgeleiteten Rh-Phosphonit-Komplex beschrieben. Erhält man unter traditioneller Verwendung von I mit R = R¹ für die Enantioselektivität den *ee*-Wert x % bzw. unter traditioneller Verwendung eines Analogons I mit R = R² den *ee*-Wert y%, dann ergibt die Verwendung eines Gemisches aus beiden Liganden eine höhere Enantioselektivität mit dem *ee*-Wert z%, d. h., es gilt z > x sowie z > y. Diese Gesetzmäßigkeit gilt allerdings nicht für beliebige Mischungen. Vielmehr wird die erhöhte Enantioselektivität immer dann beobachtet, wenn die richtige Mischung gewählt bzw. die richtige Waht der R-Reste getroffen wird. Dies ist rasch möglich, indem z. B. Kombinationen von unterschiedlichen chiralen Phosphoniten, z. B. vom Typ I, als Mischungen getestet werden. In der Mischung können auch mehr als zwei unterschiedliche chirale Liganden, z. B. vom Typ I, verwendet werden, vorzugsweise setzt man zwei ein.

Neben Mischungen von chiralen Monophosphoniten, die aus BINOL abgeleitete Vertreter der Formel I sind, können auch Mischungen aus anderen chiralen Mono-P-Liganden eingesetzt werden. Erfindungsgemäß sind dies Mischungen aus chiralen Monophosphiten, z. B. des Typs II, oder Mischungen aus Monophosphoramiditen, z. B. des Typs III. Es können aber auch chirale Phosphane, Phosphirane, Phosphinite, Phosphorigsäuretris- und bis-amide, Phosphorsäuremono- und diamide sowie Phosphorigsäurediesterfluoride in einer Mischung mit mindestens einer Verbindung der Formel B, C oder D eingesetzt werden, um nur einige zu nennen. Tatsächlich kommt für eine solche Mischung jede chirale Verbindung mit einem P-Atom in Frage. In der Praxis gibt es eine Fülle von möglichen P-Liganden, deren zentrales Gerüst durch die nachfolgende Formel IV dargestellt ist.

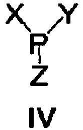

Dabei können die Atome X,Y und Z jeweils unabhängig voneinander aus der Reihe Kohlenstoff (C), Stickstoff (N), Sauerstoff (O), Schwefel (S) oder Halogen (F, Cl, Br, I) sein. An die Atome X,Y und Z sind, entsprechend ihrer Anzahl von freien Valenzen, unabhängig voneinander weitere Atome oder Gruppen von Atomen gebunden, wie z. B. bei den schon erwähnten Beispielen I, II und III. X, Y und Z können untereinander auch durch die gebundenen Atome oder Gruppen von Atomen verbrückt sein, wobei X-P-Y auch Teil eines aromatischen Systems sein kann, wobei dann X durch eine Doppelbindung an P gebunden ist und der Substituent Z entfällt.

Beispielhaft seien folgende Kombinationen von Substituenten an IV genannt:
a) X = Y = Z = C
b) X = Y = C; Z = N
c) X = Y = C; Z = O
d) X = Y = C; Z = S
e) X = Y = C; Z = Halogen (F, Cl, Br oder I)
f) X = C; Y = Z = N
g) X = C; Y = Z = O
h) X = C; Y = Z = S
i) X = C; Y = N; Z = O
j) X = Y = Z = N
k) X = Y = N; Z = O
l) X = Y = N; Z = S
m) X = Y = N; Z = Halogen (F, Cl, Br oder I)
n) X = N; Y = Z = O
o) X = N; Y = Z = S
p) X = N; Y = O; Z = Halogen (F, Cl, Br oder I)
q) X = Y = Z = O
r) X = Y = O; Z = Halogen (F, Cl, Br oder I)
s) X = Y = Z = S

Weitere Beispiele für Vertreter sind Analoga von I, II und III, bei denen der axial chirale Baustein BINOL durch Derivate, substituierte Biphenole oder durch andere chirale Diole ersetzt ist. Konkrete Vertreter sind z.B. 5,5'-Dichlor-6,6'-dimethoxy-2,2'-biphenol, Hydrobenzoin, TADDOL und aus Kohlenhydraten abgeleitete Diole. Hierbei handelt es sich jedoch nur um mögliche Beispiele, deren Erwähnung keinesfalls die Breite der Möglichkeiten einschränkt. Da die Liganden IV modular aufgebaut sind, bedeutet dies, dass die jeweiligen Bausteine z. B. chirale Alkohole, chirale Diole, chirale Amine, chirale Diamine oder chirale Aminoalkohole sind, um nur die wichtigsten Möglichkeiten zu erwähnen. Bei den nachstehenden Beispielen ist die absolute Chiralität zeichnerisch nicht angegeben. Es versteht sich von selbst, dass jeder Ligand in jeder möglichen Konfiguration eingesetzt werden kann. Die Liganden werden in enantiomerenreiner oder angereicherter Form verwendet. Vorzugsweise werden enantiomerenreine Liganden benutzt.

Die Erfindung betrifft somit auch einen chiralen Übergangsmetall-Katalysator der zuvor genannten Art, wobei die Mono-Phosphor-Liganden Phosphane, Phosphite, Phosphonite, Phosphinite, Phosphorigsäuretrisamide, Phosphorigsäuremonoesterdiamide, Phosphorigsäurediesteramide, Phosphonigsäurediamide, Phosphinigsäureamide, Phosphonigsäuremonoesteramide, Phosphorigsäurehalogenide, Phosphorigsäurediamidhalogenide, Thiophosphite, Thiophosphorigsäuretriester, Thiophosphorigsäuremonoesterdiamide oder Thiophosphorigsäurediesteramide sind.

Typische Beispiele für chirale Liganden **IVa** sind **V** und **VI,** wobei es sich um zentrale bzw. axiale Chiralität handelt:

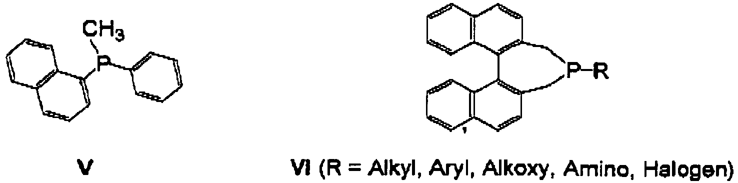

Typische Beispiele für chirale Liganden **IVb** sind **VII** und **VIII:**

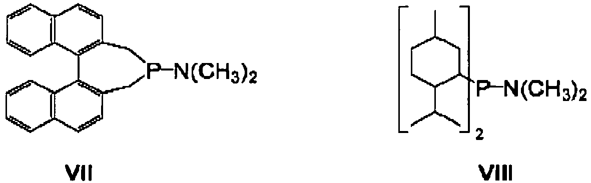

Typische Beispiele für chirale Liganden **IVc** sind **IX** und X:

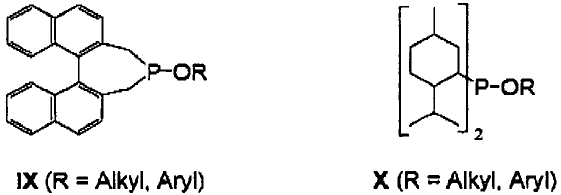

Typische Beispiele für chirale Liganden **IVd** sind die Thio-Analoga von **IX** und **X.**

Typische Beispiele für chirale Liganden **IVe** sind **XI** und **XII:**

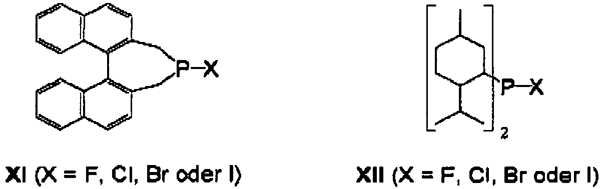

Typische Beispiele für chirale Liganden **IVf** sind **VIII** und **XIV:**

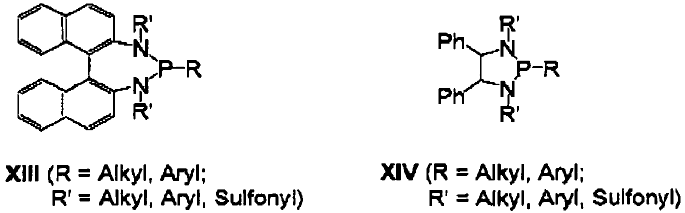

Typische Beispiele für chirale Liganden **IVg** und **IVh** sind Verbindungen I bzw. die Thio-Analoga.

Typische Beispiele für chirale Liganden **IVi** sind **XV** und **XVI:**

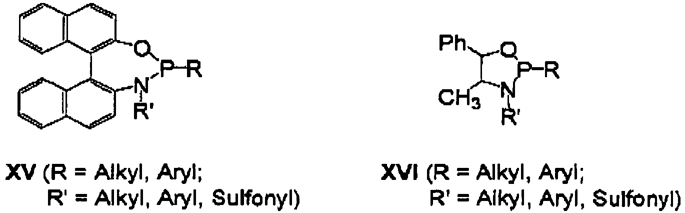

Typische Beispiele für chirale Liganden **IVJ** sind **XVII** und **XVIII:**

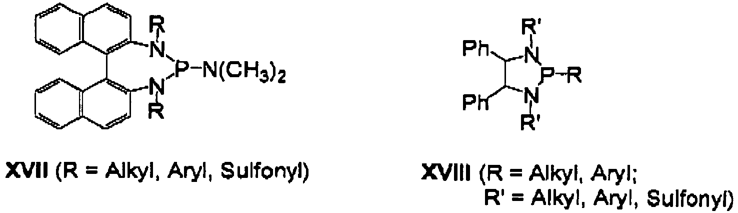

Typische Beispiele für chirale Liganden IVk sind **XIX** und **XX**:

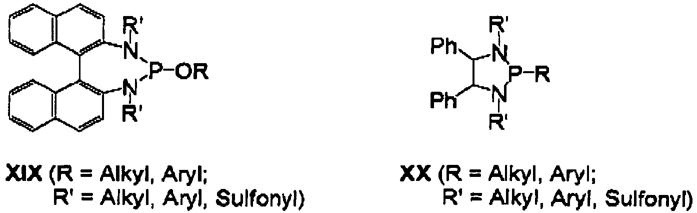

Typische Beispiele für chirale Liganden **IVI** sind die Thio-Analoga von **XIV** und **XX.**

Typische Beispiele für chirale Liganden **IVm** sind **XXI** und **XXII:**

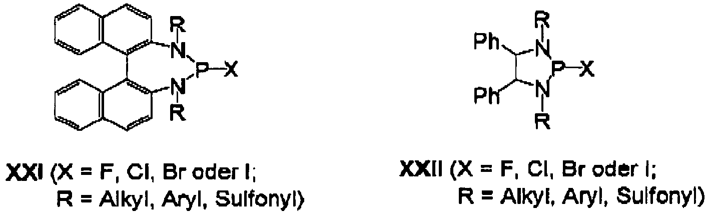

Typische Beispiele für chirale Liganden **IVn** und **IVo** sind **III** bzw. die Thio-Analoga von III.

Typische Beispiele für chirale Liganden IVp sind **XXIII** und **XXIV:**

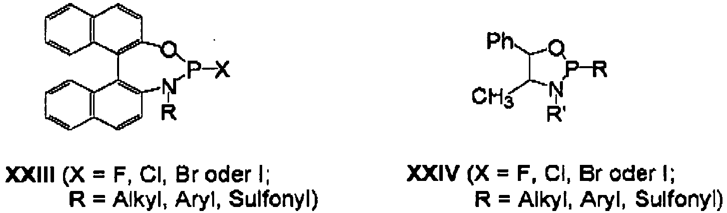

Typische Beispiele für chirale Liganden **IVg** und **IVs** sind **II** bzw. die Thio-Analoga von **II.**

Typische Beispiele für chirale Liganden IVr sind **XXV** und **XXVI:**

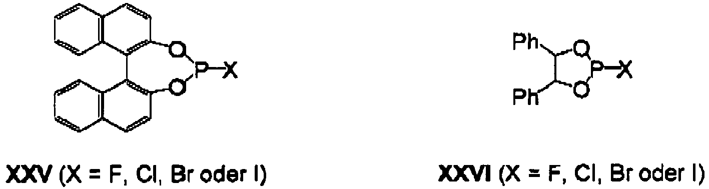

Das zugrunde liegende Prinzip der Erfindung gilt somit nicht nur dann, wenn die chiralen P-Liganden zur gleichen Substanzklasse der Verbindungen der Formel B. C oder D gehören. Eine Steigerung der Enantioselektivität wird auch dann beobachtet, wenn es sich um die Mischung von mindestens zwei chiralen strukturell unterschiedlichen Mono-Phosphor-Liganden, ausgewählt aus Mono-P-Verbindungen vom Typ **B, C** oder **D,** handelt,

Somit betrifft die Erfindung auch einen erfindungsgemäßen chiralen Übergangsmetall-Katalysator, wobei mindestens zwei Mono-Phosphor-Liganden chiral sind

Die Erfindung umfasst besonders einen erfindungsgemäßen chiralen Übergangsmetall-Katalysator, wobei als chirale Mono-Phosphor-Liganden mindestens zwei Liganden des Typs 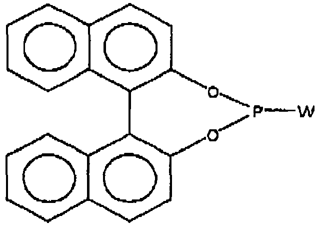 verwendet werden, wobei W unabhängig voneinander CH₃, C(CH₃)₃, *c*-C₈H₁₁ oder OCH₃ ist.

Eine zweite Variante der Erfindung beinhaltet ebenfalls die Mischung von zwei verschiedenen P-Liganden, wobei der eine (wie bisher beschrieben) ein Mono-Phosphor-Ligand, ausgewählt aus Mono-P-Verbindungen vom Typ **B, C** oder **D** Ist , der andere jedoch schiral ist. Die Verwendung einer richtigen Kombination aus einem solchen chiralen Liganden vom Typ **B, C** oder **D** und einem achiralen Analogon **IV** führt bei der Übergangsmetall-Katalyse auch in solchen Fällen überraschenderweise zu einer höheren Enantioselektivität als bei der Verwendung des relevanten chiralen Liganden allein. In manchen Fällen kann eine solche Kombination sogar verwendet werden, um die Richtung der Enantioselektivität umzukehren.

Die achiralen P-Liganden lassen sich ebenfalls entsprechend durch die allgemeine Formel IV beschreiben. Es handelt sich also z. B. um achirale Phosphane, Phosphinite, Phosphonite, Phosphorigsäuretris- und bisamide, Phosphorsäuremono- und diamide, um nur einige zu nennen. Auch Vertreter mit X = Y = Z = Halogen, also PF₃, PCl₃, PBr₃ oder Pl₃, sowie Thio-phosphite P(SR)₃, Phosphinoxide, Phosphinsulfide, Iminophosphorane, Phosphirane und Phosphinine kommen in Frage.

Ein solcher achiraler Ligand kann eine Mono-P-Verbindung vom Typ H-T sein: 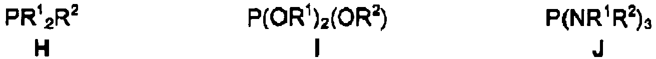 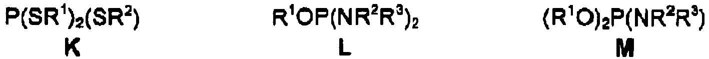 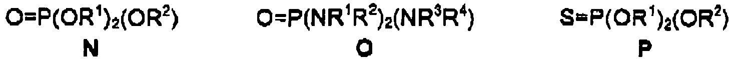 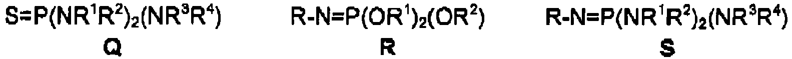 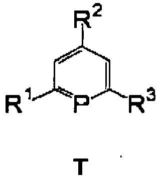 wobei die Reste R¹, R², R³ und R⁴ unabhängig voneinander aus der Reihe Wasserstoff, Halogen, gesättigte und ungesättigte lineare und verzweigte C₁-C₅₀ Alkyl-, C₈-C₅₀ Aryl-, C₁-C₅₀ Heteroaryl-, Alkinyl-, Silyl-, Nitro-, Nitril-, Ester-, Carboxyl-, Carbonyl-, Amid- und Selenidgruppen ausgewählt sind,
wobei R¹, R², R³ und R⁴ ihrerseits weitere Substituenten tragen, funktionalisiert oder verbrückt sein können.

Was die Darstellung der Katalysatoren bzw. Präkatalysatoren angeht, so kommt die in der Literatur bekannte Vorgehensweise zum Tragen, die üblicherweise zur Darstellung von traditionellen Homokombinationen M(L^{a})ₙ verwendet wird. Dies bedeutet, dass die jeweilige Liganden-Mischung mit einem geeigneten Übergangsmetall-Komplex zusammengebracht wird. Die Übergangsmetall-Komplexe können gängige Salze wie MXₙ (X = F, Cl, Br, I, BF₄, ClO₄, RCO₂, RSO₃, acac) sein, z. B. [Rh(OAc)₂]₂, Rh(acac)₃, Cu(CF₃SO₃)₂, CuBF₄, Ag(CF₃SO₃), Au(CO)Cl, In(CF₃SO₃)₃, Fe(ClO₄)₃, NiCl₂(COD) (COD = 1,5-Cyclooctadien), Pd(OAc)₂, [C₃H₅PdCl]₂, PdCl₂(CH₃CN)₂ oder La(CF₃SO₃)₃, um nur einige zu nennen. Es kann sich aber auch um Metall-Komplexe handeln, die u. a. Liganden wie Olefine, Diene, Pyridin, CO oder NO tragen (um nur einige zu nennen). Letztere werden durch die Reaktion mit den P-Liganden ganz oder teilweise verdrängt. Kationische Metall-Komplexe können ebenfalls eingesetzt werden. Die Fachwelt kennt eine Vielzahl von Möglichkeiten (G. Wilkinson, Comprehensive Coordination Chemistry, Pergamon Press, Oxford (1987); B. Cornils, W. A. Herrmann, Applied Homogeneous Catalysis with Organometallic Compounds, VCH, Weinheim (1996)). Gängige Beispiele sind Rh(COD)₂BF₄, [(Cymol)RuCl₂]₂, (Pyridin)₂Ir(COD)BF₄, Ni(COD)₂, (TMEDA)Pd(CH₃)₂ (TMEDA = N, *N*, *N*', *N'*-Tetramethylethylendiamin), Pt(COD)₂, PtCl₂(COD) oder [RuCl₂(CO)₃]₂, um nur einige wenige zu nennen. Zu den Metallen zählen Rh, Ir, Ru, Ni, Pd oder Pt..

Zur Illustration und zum Nachweis, dass es sich um strukturell neuartige Katalysatoren handeit, seien die Umsetzungen von Rh(COD)₂BF₄ mit den reinen (*R*)-konfigurierten Phosphoni-ten I (R = CH₃) und **I** (R = C(CH₃)₃) unter Bildung der traditionellen Rh-Komplexe **XXVII** bzw. **XXVIII** und die Umsetzung mit einer 1 : 1 Mischung aus beiden Liganden unter Bildung des "gemischten" Komplexes **XXIX** (neben den Komplexen **XXVII** und **XXVIII)** erwähnt. Die ¹H-, ¹³C- und ³¹P-NMR Spektren des Komplexes **XXIX** sind charakteristisch für eine "gemischte" Verbindung, d. h. sie unterscheiden sich von den Spektren der konventionellen Komplexe **XXVII** und **XXVIII.** Isoliert man das Gemisch der Komplexe **XXVII, XXVIII** und **XXIX,** so lassen sich massenspektrometrisch (ESI-MS) alle drei Komplexe eindeutig nachweisen, wobei **XXIX** die Hauptkomponente ist. Was die praktische Anwendung angeht, so muss der "Misch-Komplex" **XXIX** nicht unbedingt von den reinen Komplexen **XXVII** und **XXVIII** getrennt werden, denn es zeigt sich anhand kinetischer Untersuchungen, dass die Mischung aus den drei Katalysatoren aktiver ist als die jeweiligen Homokombinationen **XXVII** und **XXVIII.** Analoge NMR und ESI-MS Untersuchungen von anderen "gemischten" Metall-Katalysatoren (Heterokombinationen) beweisen ebenfalls die einzigartige Struktur dieser Komplexe und belegen, dass es sich um eine neue Substanzklasse handelt.

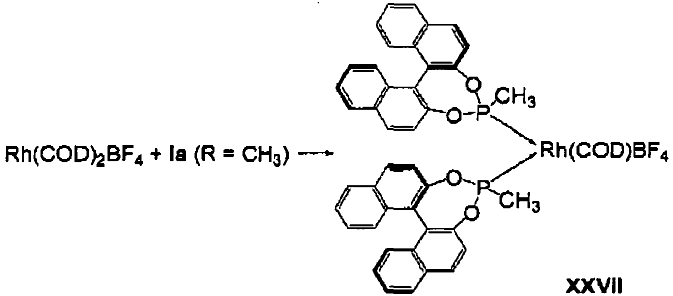

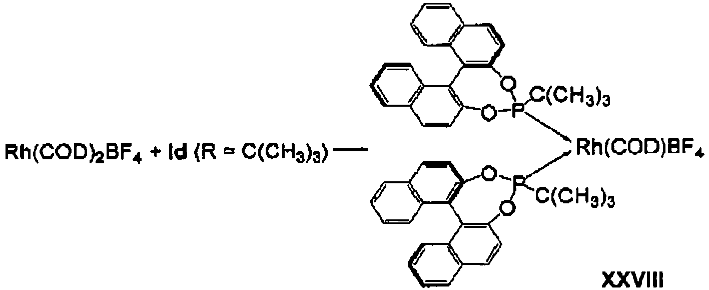

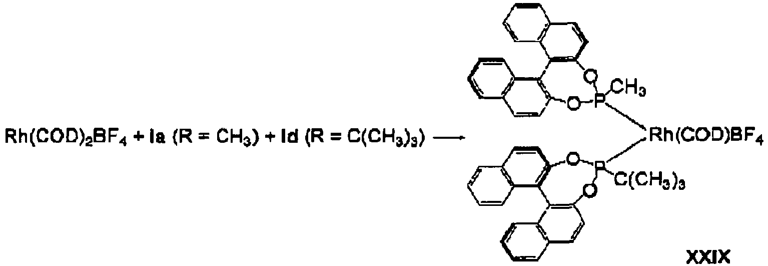

Entscheidend für die Erfindung ist der unerwartete Befund, dass sich das gesamte Katalyse-Profil der traditionellen Katalysatoren, z. B. **XXVII** und **XXVIII,** stark von dem des "gemischten" Komplexes, z. B. **XXIX,** unterscheidet. Es zeigt sich, dass z. B. bei einer Olefinhydrierung der erfindungsgemäße Katalysator **XXIX** eine deutlich höhere Enantioselektivität bewirkt als sie bei Verwendung der traditionellen Katalysatoren **XXVII** und **XXVIII** erzielt wird. Gleichzeitig wird eine höhere Reaktionsgeschwindigkeit beobachtet. Was die praktische Seite angeht, so ist deshalb eine Trennung und Reinigung des "gemischten" Katalysators **XXIX** nicht unbedingt erforderlich, d. h. das Gemisch aus **XXIX** und **XXVII/XXVIII** kann eingesetzt werden, denn die hohe Aktivität von **XXIX** bestimmt das katalytische Ergebnis. Ein weiteres typisches Beispiel bezieht sich auf die Umsetzung von Rh(COD)₂BF₄ mit einer 1:1 Mischung aus **Ic** und **IIa**, die zum Gemisch aus Rh(**Ic**)₂(COD)BF₄, Rh(**IIa**)₂(COD)BF₄ und Rh(1c)(IIa)(COD)BF₄ führt. Auch hier zeigen Untersuchungen, dass die Heterokombination andere spektroskopische Eigenschaften hat als die traditionellen Homokombinationen.

Die erfindungsgemäßen "Misch-Katalysatoren" auf der Basis von Heterokombinationen unterschiedlicher Mono-P-Verbindungen können mehrere chirale oder achirale P-Liganden enthalten, vorzugsweise sind es zwei verschiedene P-Liganden. Das Verhältnis der P-Liganden kann relativ zueinander im Metall-Komplex beliebig variiert werden. Handelt es sich z. B, um zwei verschiedene Liganden A und B, so kann das relative Verhältnis A : B vorzugsweise zwischen 1 : 4 und 4 : 1 variiert werden, besonders vorzugsweise wählt man ein A : B Verhältnis von ca. 1 : 1. Das Verhältnis von Metall zu Substrat bewegt sich im üblichen Rahmen, d. h. zwischen 1 : 5 und 1 : 1,000.000.

Durch das Durchsuchen von Bibliotheken von Mischungen aus zwei chiralen Mono-P-Liganden oder von Mischungen aus einem chiralen und einem achiralen P-Liganden besteht die einfache Möglichkeit, den besten Misch-Katalysator (Heterokombination) für eine gegebene Übergangsmetall-katalysierte Stoffumwandlung zu finden. Diese Vorgehensweise ist einfach und mit modernen Geräten, die in der kombinatorischen Katalyse üblich sind, rasch zu vollziehen. Dazu gehören parallelisierte Reaktoren sowie Pipettier-Roboter (M. T. Reetz, Angew. Chem., 113, 292 (2001)). Man kann aber auch sequentiell vorgehen, d.h. eine Mischung nach der anderen testen. Die erfindungsgemäße Verwendung von zwei (oder mehreren) chiralen Mono-P-Liganden oder von Mischungen aus chiralen und achiralen Mono-P-Liganden gilt für alle Übergangsmetall-katalysierten Reaktionen (E. N. Jacobsen, A. Pfaltz, H. Yamamoto, Comprehensive Asymmetric Catalysis, Vol. I-III, Springer, Berlin (1999)), insbesondere für asymmetrische Hydrierungen, Hydroformylierungen, Hydroborierungen, Hydrosilylierungen, Hydrovinylierungen, Hydroaminierungen, Epoxidierungen, Hydroxylierungen, Aminohydroxylierungen, Substitutionen (z. B. Allylsubstitutionen), Heck-, Stille-, Suzukl- und Negishi-Kupplungen, Michael-Additionen, Aldol-Additionen, Diels-Alder-Reaktionen, Cyclopropanierungen, CH-Einschiebungsreaktionen und 1,3-dipolare Cycloadditionen.

### Beispiele

### Beispiel 1: Rh-katalysierte Hydrierung von N-Acylacrylsäuremethylester unter Verwendung von Liganden des Typs I, II und III.

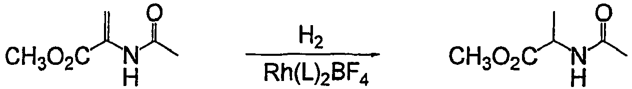

In einem ausgeheizten 50 ml Schlenk-Gefäß unter Argon-Atmosphäre wurden eine Mischung aus 0.6 ml einer 1.7 mM Lösung des ersten Liganden und 0.6 ml einer 1.7 mM Lösung des zweiten Liganden in abs. Dichlormethan vorgelegt. Diese Lösung wurde mit 0.5 ml einer 2.0 mM Lösung von [Rh(COD)₂]BF₄ in Dichlormethan versetzt und 5 Minuten bei Raumtemperatur gerührt. Anschließend wurden 9 ml einer 0.112 M Lösung des Substrates in Dichlormethan zugegeben. Es wurde dreimal bis zum leichten Sieden des Lösungsmittels evakuiert und mit Wasserstoff begast. Bei 1.3 bar Wasserstoffdruck wurde für die Dauer der Reaktion gerührt. Die Umsatzbestimmung erfolgte nach Verdünnung der Reaktionslösung gaschromatographisch. Für die Bestimmung des Enantiomerenüberschusses wurden ca. 1.5 ml der Reaktionslösung adsorptiv über wenig Kieselgel filtriert und gaschromatographisch oder mittels HPLC untersucht. Die Versuche wurden mit 20 Gefäßen parallel durchgeführt.

Zum Vergleich wurden die reinen Liganden unter sonst gleichen Bedingungen bei der Rhkatalysierten Hydrierung getestet. Die Ergebnisse sind in Tabelle 1 zusammengefasst. Danach sind gleich mehrere Mischkatalysatoren (Heterokombinationen) deutlich enantioselektiver (z. B. Einträge 16, 17, 40, 42, 44 und 45) als die aus traditionellen reinen Liganden aufgebauten Analoga (Einträge 1 - 14).

**Tabelle 1. Rh-katalysierte Hydrierung von IVa^{[a]}**

| Eintrag | Liganden | *ee* [%] (Konfig.) |
|---|---|---|
| | **Homokombinationen** | |
| 1 | (*R*)**Ia**/(*R*)**Ia** | 91.8 (*S*) |
| 2 | (*R*)**Ib**/(*R*)**Ib** | 94.4 (S) |
| 3 | (*R*)**Ic**/(*R*)**Ic** | 92.0 (*S*) |
| 4 | (*R*)**Id**/(*R*)**Id** | 93.3 (*S*) |
| 5 | (*R*)**Ie**/(*R*)**Ie** | 72.8 (*S*) |
| 6^{[b]} | (*R*)**If**/(*R*)**If** | 7.4 (*S*) |
| 7 | (*S*)**IIa**/(*S*)**IIa** | 76.6 (*R*) |
| 8 | (*S*)**IIb**/(*S*)**IIb** | 83.6 (*R*) |
| 9 | (*R*)**IIc**/(*R*)**IIc** | 94.6 (*S*) |
| 10 | (*S*)**IId**/(*S*)**IId** | 95.4 (*R*) |
| 11^{[c]} | (*S*)**IIe**/(*S*)**IIe** | 78.6 (*R*) |
| 12^{[d]} | (*S*)**IIf**/(*S*)**IIf** | 32.4 (R) |
| 13 | (*S*)**IIg**/(*S*)**IIg** | 94.4 (R) |
| 14 | (*S*)**IIh**/(*S*)**IIh** | 92.4 (R) |
| | | |
| | **Heterokombinationen** | |
| 15 | (*R*)**Ia**/(*R*)**Ib** | 92.6 (*S*) |
| 16 | (*R*)**Ia**/(*R*)**Ic** | 97.9 (*S*) |
| 17 | (*R*)**Ia**/(*R*)**Id** | 97.8 (*S*) |
| 18 | (*R*)**Ic**/(*R*)**Id** | 94.1 (*S*) |
| 19 | (*R*)**Id**/(*R*)**Ie** | 75.8 (*S*) |
| 20 | (*R*)**Id**/(*R*)**If** | racemisch |
| 21 | (*R*)**IIa**/(*R*)**IIb** | 80.0 (*S*) |
| 22 | (*R*)**IIa**/(*R*)**IIc** | 76.6 (*S*) |
| 23 | (*R*)**IIa**/(*R*)**IId** | 89.0 (*S*) |
| 24 | (*R*)**IIa**/(*R*)**IIe** | 77.4 (*S*) |
| 25 | (*R*)**IIa**/(*R*)**IIf** | 84.6 (*S*) |
| 26 | (*R*)**IIa**/(*R*)**IIg** | 87.2 (*S*) |
| 27 | (*R*)**IIb**/(*R*)**IIc** | 79.0 (*S*) |
| 28 | (*R*)**IIb**/(*R*)**IId** | 91.2 (*S*) |
| 29 | (*R*)**IIb/**(*R*)**IIe** | 80.8 (*S*) |
| 30 | (*R*)**IIb**/(*R*)**IIg** | 90.0 (*S*) |
| 31 | (*R*)**IId**/(*R*)**IIc** | 94.2 (*S*) |
| 32 | (*R*)**IId**/(*R*)**IIe** | 92.2 (*S*) |
| 33 | (*R*)**IIe**/(*R*)**IIc** | 73.6 (*S*) |
| 34 | (*R*)**IIg**/(*R*)**IIc** | 94.6 (*S*) |
| 35 | (*R*)**IIg**/(*R*)**IId** | 94.8 (*S*) |
| 36 | (*R*)**IIg**/(*R*)**IIe** | 91.2 (*S*) |
| 37 | (*R*)**Ia**/(*R*)**IIa** | 81.9 (*S*) |
| 38 | (*R*)**Ia**/(*R*)**IIc** | 94.4 (*S*) |
| 39 | (*R*)**Ia**/(*R*)**IId** | 93.0 (*S*) |
| 40 | (*R*)**Ic**/(*R*)**IIa** | 96.4 (*S*) |
| 41 | (*R*)**Ic**/(*R*)**IId** | 91.8 (*S*) |
| 42 | (*R*)**Id**/(*R*)**IIa** | 98.0 (*S*) |
| 43 | (*R*)**Id**/(*R*)**IIc** | 94.6 (*S*) |
| 44 | (*R*)**Id**/(*R*)**IIh** | 97.2 (*S*) |
| 45 | (*R*)**Ic**/(*R*)**IIh** | 95.6 (*S*) |

| | | |
|---|---|---|
| ^{[a]} Rh/Substrat-Verhältnis 1 : 1000; Rh/P-Verhältnis 1 : 2; Solvens: CH₂Cl₂; p(H₂): 1.3 bar; T: 20 °C; Reaktionszeit: 20 h; Umsatz: 100%. ^{[b]} Umsatz: 1%. ^{[c]} Umsatz: 93%. ^{[d]} Umsatz: 62%. | | |

### Beispiel 2: Rh-katalysierte Hydrierung von Phenyl-N-acylacrylsäuremethylester unter Verwendung von Liganden des Typs I.

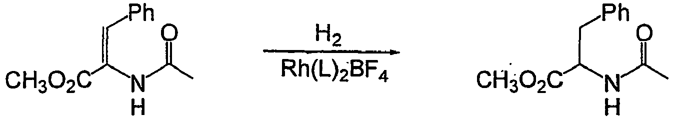

In einem ausgeheizten 50 ml Schlenk-Gefäß unter Argon-Atmosphäre wurde eine Mischung aus 0.6 ml einer 1.7 mM Lösung des ersten Liganden und 0.6 ml einer 1.7 mM Lösung des zweiten Liganden in abs. Dichlormethan vorgelegt. Diese Lösung wurde mit 0.5 ml einer 2.0 mM Lösung von [Rh(COD)₂]BF₄ in Dichlormethan versetzt und 5 Minuten bei Raumtemperatur gerührt. Anschließend wurden 9 ml einer 0.112 M Lösung des Substrates in Dichlormethan zugegeben. Es wurde dreimal bis zum leichten Sieden des Lösungsmittels evakuiert und mit Wasserstoff begast. Bei 1.3 bar Wasserstoffdruck wurde für die Dauer der Reaktion gerührt. Die Umsatzbestimmung erfolgte nach Verdünnung der Reaktionslösung gaschromatographisch. Für die Bestimmung des Enantiomerenüberschusses wurden ca. 1.5 ml der Reaktionslösung adsorptiv über wenig Kieselgel filtriert und gaschromatographisch oder mittels HPLC untersucht. Die Versuche wurden mit 20 Gefäßen parallel durchgeführt.

Die gemessenen Enantioselektivitäten bei 100% Umsatz sind wie folgt: (*R*)**Ia**/(*R*)**Ic**: *ee* = 96.7% (*S*); (*R*)**Ia**/(*R*)**Id**: *ee* = 99.2% (*S*); (*R*)**Ib**/(*R*)**Id**: *ee* = 94.6% (*S*) gegenüber (*R*)**Ia**/(*R*)**Ia**: *ee* = 89.9% (*S*); (*R*)**Ib**/(*R*)**Ib**: *ee* = 89.2% (*S*); (*R*)**Id**/(*R*)**Id**: *ee* = 69.1% (*S*).

### Beispiel 3: Rh-katalysierte Hydrierung von 1-N-Acylaminostyrol unter Verwendung von Liganden des Typs I und II.

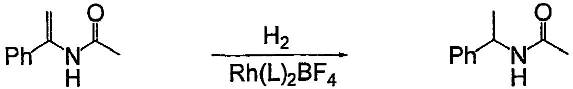

Analog zur Vorschrift in Beispiel 1 wurde eine Mischung aus 0.5 ml einer 2 mM Lösung von Rh(COD)₂BF₄ in trockenem CH₂Cl₂, 0.25 ml einer 4 mM Lösung eines Phosphonits I und 0.25 ml einer 4 mM Lösung eines zweiten Phosphonits **I** in CH₂Cl₂ hergestellt. Dies führte zur Änderung der Farbe von orange nach gelb. Nach der Zugabe von 1-*N*-Acylaminostyrol (0.5 mM) in 1 ml CH₂Cl₂ wurde die Reaktionslösung 22 Stunden bei 30 °C und 1.5 bar H₂-Druck gerührt. Die GC-Analyse gibt den Umsatz und den ee-Wert an.

### Rh-katalysierte Hydrierung von 1-N-Acetamidostyrol

| Eintrag | Liganden | ee (%) Konfiguration |
|---|---|---|
| | Homokombinationen | |
| 1 | **(*R*)Ia/(*R*)Ia** | 75.6 (*S*) |
| 2^{b} | **(*R*)Id/(*R*)Id** | 83.0 (*S*) |
| 3 | **(*R*)IIa/(*R*)IIa** | 76.0 (*S*) |
| 4 | **(*R*)IIc/(*R*)IIc** | 84.8 (*S*) |
| 5 | **(*R*)IIe/(*R*)IIe** | 85.4 (*S*) |
| 6 | **(*R*)IIi/(*R*)IIi** | 91.4 (*S*) |
| | Heterokombinationen | |
| 7^{c} | **(*R*)Ia/(*R*)Id** | 96.1 (*S*) |
| 8 | **(*R*)IIa/(*R*)Id** | 95.0 (*S*) |
| 9 | **(*R*)IIe/(*R*)IIc** | 88.6 (*S*) |
| 10 | **(*R*)IIi/(*R*)Id** | 97.4 (*S*) |

| | | |
|---|---|---|
| ^{[a]}Rh/Substrat- Verhältnis 1: 500, Rest Tabelle 1 ^{[b]} Umsatz: 13% ^{[c]} Umsatz: >95% | | |

### Beispiel 4: Rh-katalysierte Hydrierung von 1-N-Acylamino-1-p-chlor-phenylethylen.

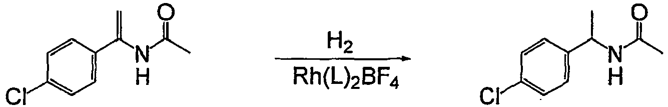

Die Hydrierungen erfolgen analog zur Vorschrift in Beispiel 3. Die gemessenen Enantioselektivitäten bei > 95% Umsatz sind wie folgt: **(*R*)Ia/(*R*)Id:** *ee* = 95.0% (*S*) gegenüber (*R*)**Ia**(*R*)**Ia**: *ee* = 73.0% (*S*) und (*R*)**Id**/(*R*)**Id**: *ee* = 16.2% (*S*), Umsatz 79%.

### Beispiel 5: Variation des Verhältnisses der beiden P-Liganden Ia und Id bei der Rhkatalysierten Hydrierung von 1-N-Acylaminostyrol.

Die Hydrierungen wurden wie in Beispiel 3 durchgeführt, jedoch unter Variation des relativen Verhältnisses von **Ia** und **Id.** Dabei wurde das Rh : P-Verhältnis bei 1 : 2 und das Rh : Substrat-Verhältnis bei 1 : 500 konstant gehalten. Die mittels GC bestimmten Enantioselektivitäten bei Umsätzen von > 95% sind in Tabelle 2 zusammengefasst.

**Tabelle 2.**

| Einfluss des Verhältnisses **Ia** : **Id** auf die Enantioselektivität der Hydrierung von **VIa**^{[a]} | | |
|---|---|---|
| Eintrag | Liganden (*R*)**Ia**/(*R*)**Id** | *ee* [%] (Konfig.) |
| 1^{[b]} | 1 : 5 | 95.4 (*S*) |
| 2 | 1 : 3 | 97.4 (*S*) |
| 3 | 1 : 2 | 97.2 (*S*) |
| 4 | 1 : 1 | 96.4 (*S*) |
| 5 | 2 : 1 | 88.8 (*S*) |
| 6 | 3 : 1 | 85.0 (*S*) |
| 7 | 5 : 1 | 81.2 (*S*) |

| | | |
|---|---|---|
| ^{[a]} In allen Fällen betrug das Rh/((*R*)**Ia**/(*R*)**Id**)-Verhältnis 1 : 2 und das Rh/Substrat-Verhältnis 1 : 500. Solvens: CH₂Cl₂; p(H₂): 1.5 bar; T: 20 °C; Reaktionszeit: 1 h; Umsatz: 100%. ^{[b]} Umsatz: 95%. | | |

### Beispiel 6: Rh-katalysierte Hydrierung von 1-N-Acylamino-1-naphthylethylen unter Verwendung von Liganden des Typs I.

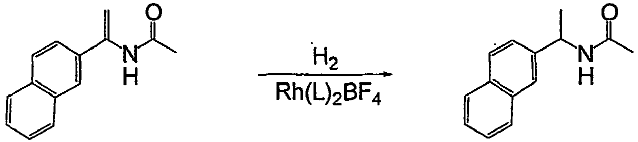

Die Hydrierungen erfolgten analog zur Vorschrift in Beispiel 3. Die gemessenen Enantioselektivitäten bei > 95% Umsatz sind wie folgt: (*R*)**Ia**/(*R*)**Id**: *ee* = 97.0% (*S*) gegenüber (*R*)**Ia**/(*R*)**Ia**: *ee* = 78.2% (*S*) und (*R*)**Id**/(*R*)**Id**: *ee* = <3% (*S*), Umsatz 35%.

### Beispiel 7: Rh-katalysierte Hydrierung von Itaconsäuredimethylester unter Verwendung von Liganden des Typs I.

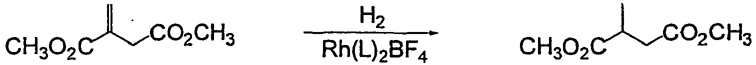

Die Hydrierung erfolgte analog zur Vorschrift in Beispiel 1. Die mittels GC bestimmten Enantioselektivtäten bei quantitativem Umsatz sind in Tabelle 3 zusammengefasst.

**Tabelle 3. Rh-katalysierte Hydrierung von VIII^{[a]}**

| Eintrag | Liganden | *ee* [%] (Konfig.) |
|---|---|---|
| | **Homokombinationen** | |
| 1 | (*R*)**Ia**/(*R*)**Ia** | 90.2 (*R*) |
| 2 | (*R*)**Ib**/(*R*)**Ib** | 71.4 (*R*) |
| 3 | (*R*)**Ic**/(*R*)**Ic** | 21.9 (*R*) |
| 4 | (*R*)**Id**/(*R*)**Id** | 57.3 (*R*) |
| 5 | (*R*)**Ie**/(*R*)**Ie** | 28.8 (*R*) |
| | | |
| | **Heterokombinationen** | |
| 6 | (*R*)**Ia**/(*R*)**Ib** | 82.4 (*R*) |
| 7 | (*R*)**Ia**/(*R*)**Ic** | 88.6 (*R*) |
| 8 | (*R*)**Ia**/(*R*)Id | 96.4 (*R*) |
| 9 | (*R*)**Ib**/(*R*)**Id** | 92.2 (*R*) |
| 10 | (*R*)**Ic**/(*R*)**Id** | 69.1 (*R*) |
| 11 | (*R*)**Ic**/(*R*)**Ie** | 50.0 (*R*) |
| 12 | (*R*)**Id**/(*R*)**Ie** | 57.4 (*R*) |

| | | |
|---|---|---|
| ^{[a]} In allen Fällen betrug das Rh/P-Verhältnis 1 : 2 und das Rh/Substrat-Verhältnis 1 : 1000. Solvens: CH₂Cl₂; p(H₂): 1.3 bar; T: 20 °C; Reaktionszeit: 20 h; Umsatz: 100%. | | |

Die quantitative Hydrierung kann auch bei Reduzierung des Rh/Substrat-Verhältnisses unter sonst gleichen Bedingungen durchgeführt werden. Bei Rh : Substrat = 1 : 6000 beträgt der ee-Wert 95.8%(*R*); bei 1 : 10.000 beträgt der ee-Wert 95.4%(*R*); bei 1 : 20.000 beträgt der ee-Wert 94.6%(*R*).

### Beispiel 8: Rh-katalysierte Hydrierung von N-Acylaminoacrylsäuremethylester unter Verwendung von chiralen Phosphoniten I, Phosphiten II, Phosphoramiditen III und achiralen Mono-P-Liganden.

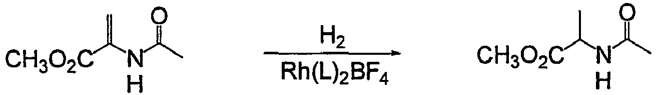

Die Bedingungen der Hydrierungen wurden wie in Beispiel 1 gewählt, wobei ein chiraler (Verbindung **I** oder **II**) und ein achiraler P-Ligand (Verbindung **XXX** oder **XXXI**) im Verhältnis von 1 : 1 eingesetzt wurden.

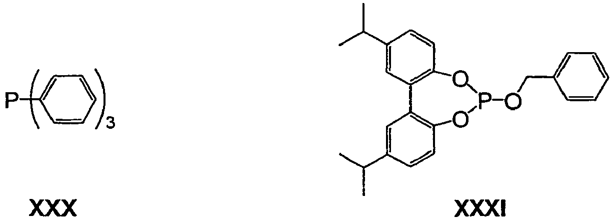

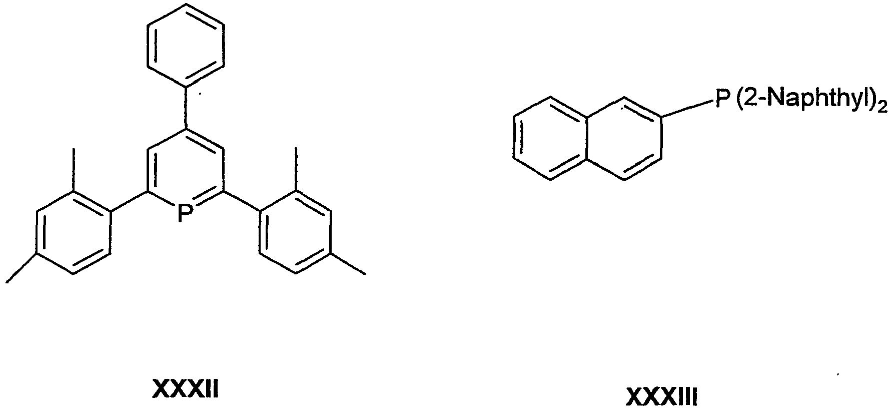

Die Ergebnisse der Hydrierung unter Umkehr der Enantioselektivität bei quantitativem Umsatz sind wie folgt:

| Eintrag | Liganden | ee (%) Konfiguration |
|---|---|---|
| 1 | **(*R*)Ia/XXX** | 19.6 (*R*) |
| 2 | **(*R*)IIa/XXX** | 10.0 (*R*) |
| 3 | **(*R*)IId/XXX** | 45.4 (*R*) |
| 4 | **(*R*)Ia/XXXII** | 58.6 *(R)* |
| 5 | **(*R*)Id/XXXII** | 52.6 (*R*) |
| 6 | **(*R*)IId/XXXIII** | 34.8 (*R*) |

| | | |
|---|---|---|
| Rh/Substrat-Verhältnis 1 : 1000; Rh/P-Verhältnis 1 : 2; Solvens: CH₂Cl₂; p(H₂): 1.3 bar; T: 20 °C; Reaktionszeit: 20 h; Umsatz: 100%. | | |

### Beispiel 9: Herstellung und Charakterisierung des Katalysatorsystems Rh[Ia][Id][COD]BF₄ + Rh[Ia]₂[COD]BF₄ + Rh[Id]₂[COD]BF₄.

Die Mischung von (*R*)**Ia** (13.2 mg; 0.04 mmol) und (*R*)**Id** (14.9 mg; 0.04 mmol) in CD₂Cl₂ (1 ml) wurde mit Rh[COD]₂BF₄ (16.2 mg; 0.04 mmol) in CD₂Cl₂ (1 ml) behandelt. Die ¹H-, ¹³C- und ³¹P-NMR Spektren zeigen die Anwesenheit der beiden Homokombinationen Rh[(*R*)**Ia**]₂[COD]BF₄ (**XXVII**) und Rh[(*R*)**Id**]₂[COD]BF₄ (**XXVIII**) und der Heterokombination Rh[(*R*)**Ia**][(*R*)**Id**]BF₄ (**XXIX**) im Verhältnis von etwa 20 : 20 : 60. Die charakteristischen Peaks und Verteilungen im ³¹P-Spektrum sind wie folgt:

³¹P NMR (121.5 MHz, 223K, CD₂Cl₂, rel. ext. 85% H₃PO₄):

| | **δ_{P} (Ia)** | **δ_{P} (Id)** | ***J_{RhP}*, Hz** | ***J_{PP}*, Hz** | **%** |
|---|---|---|---|---|---|
| Rh[(*R*)**Ia**]₂[COD]BF₄ | 189.8 | | 206 | | 18 |
| Rh[(*R*)**Id**]₂[COD]BF₄ | | 204.8 | 207 | | 23 |
| Rh[(*R*)**Ia**][(*R*)**Id**][COD]BF₄ | 187.0 | 208.3 | 208,210 | 38 | 15 |
| (1. Isomer) | | | | | |
| Rh[(*R*)**Ia**][(*R*)**Id**][COD]BF₄ | 202.4, 201.8 | | 201,212 | 40 | 44 |
| (2. Isomer) | | | | | |

### Beispiel 10: Herstellung, Isolierung und Charakterisierung des Katalysatorsystems Rh[Ia][Id][COD]BF₄ + Rh[Ia]₂[COD]BF₄ + Rh[Id]₂[COD]BF₄ mittels ESI-MS.

Eine Mischung von (*R*)**Ia** (32.6 mg; 0.1 mmol) und (*R*)**Id** (36.9 mg; 0.1 mmol) in CH₂Cl₂ (20 ml) wurde bei -78°C mit Rh[COD]₂BF₄ (40.7 mg; 0.1 mmol) in CH₂Cl₂ (5 ml) versetzt. Nach Erwärmen auf Raumtemperatur wurde das Lösungsmittel auf 5 ml eingeengt und mit 15 ml Pentan ein gelber Feststoff ausgefällt. Dieser wurde dreimal mit Pentan gewaschen und im Vakuum getrocknet. Im ESI-MS-Spektrum lassen sich Fragmente der in Bespiel 9 beschriebenen Komplexe nachweisen, wobei der Komplex **XXIX** der Heterokombination die Hauptkomponente darstellt. Das ³¹P-NMR-Spektrum in CD₂Cl₂ zeigt dieselben Signale, die bereits in Beispiel 9 beschrieben und zugeordnet sind.

Rh[(*R*)**Ia**]₂[COD]BF₄ (**XXVII**):
MS(ESI/pos. in CH₂Cl₂): m/z = 763 [M⁺-BF₄-COD].

Rh[(*R*)**Id**]₂[COD]BF₄ (**XXVIII**):
MS(ESI/pos. in CH₂Cl₂): m/z = 847 [M⁺-BF₄-COD].

Rh[(*R*)**Ia**][(*R*)**Id**][COD]BF₄ (**XXIX**):
MS(ESI/pos. in CH₂Cl₂): m/z = 805 [M⁺-BF₄-COD].

### Beispiel 11: Untersuchung einer Mischung von Rh[Ia]₂[COD]BF₄ und Rh[Id]₂[COD]BF₄

Eine Lösung von Rh[**Ia**]₂[COD]BF₄ (3.3 mg; 0.0034 mmol; 0.5 ml CD₂Cl₂) und eine Lösung von Rh[**Id**]₂[COD]BF₄ (3.5 mg; 0.0034 mmol; 0.5 ml CD₂Cl₂) wurden gemischt. Das Gemisch wurde ³¹P-NMR-spektroskopisch untersucht. Es zeigt sich anhand der Signale (analog zu Beispiel 9), dass dieselben Komponenten Rh[(*R*)**Ia**]₂[COD]BF₄ **(XXVII),** Rh[(*R*)**Id**]₂[COD]BF₄ (**XXVIII**) und Rh[(*R*)**Ia**][(*R*)**Id**]BF₄ (**XXIX**) wie in Beispiel 9 vorliegen.

### Beispiel 12: Herstellung, Isolierung und Charakterisierung des Katalysatorsystems Rh[Ia][Ic][COD]BF₄ + Rh[Ia]₂[COD]BF₄ + Rh[Ic]₂[COD]BF₄ mittels ESI-MS.

Eine Mischung von (*R*)**Ia** (29.4 mg; 0.09 mmol) und (*R*)**Ic** (35.5 mg; 0.09 mmol) in CH₂Cl₂ (20 ml) wurde bei -78°C mit Rh[COD]₂BF₄ (36.5 mg; 0.09 mmol) in CH₂Cl₂ (5 ml) versetzt. Nach Erwärmen auf Raumtemperatur wurde das Lösungsmittel auf 5 ml eingeengt und mit 15 ml Pentan ein gelber Feststoff ausgefällt. Dieser wurde dreimal mit Pentan gewaschen und im Vakuum getrocknet. Im ESI-MS-Spektrum lassen sich die folgenden Fragmente nachweisen:

Rh[(*R*)**Ia**]₂[COD]BF₄:
MS(ESI/pos. in CH₂Cl₂): m/z = 763 [M⁺-BF₄-COD].

Rh[(*R*)**Ic**]₂[COD]BF₄:
MS(ESI/pos. in CH₂Cl₂): m/z = 897 [M⁺-BF₄-COD-2H].

Rh[(*R*)**Ia**][(*R*)**Ic**][COD]BF₄:
MS(ESI/pos. in CH₂Cl₂): m/z = 831 [M⁺-BF₄-COD].

### Beispiel 13: Herstellung, Isolierung und Charakterisierung des Katalysatorsystems Rh[IIa][Ic][COD]BF₄ + Rh[IIa]₂[COD]BF₄ + Rh[Ic]₂[COD]BF₄ mittels ESI-MS.

Eine Mischung von (*R*)**IIa** (44.6 mg; 0.13 mmol) und (*R*)**Ic** (51.8 mg; 0.13 mmol) in CH₂Cl₂ (20 ml) wurde bei -78°C mit Rh[COD]₂BF₄ (52.8 mg; 0.13 mmol) in CH₂Cl₂ (5 ml) versetzt. Nach Erwärmen auf Raumtemperatur wurde das Lösungsmittel auf 5 ml eingeengt und mit 15 ml Pentan ein gelber Feststoff ausgefällt. Dieser wurde dreimal mit Pentan gewaschen und im Vakuum getrocknet. Im ESI-MS-Spektrum lassen sich die folgenden Fragmente nachweisen:

Rh[(*R*)**IIa**]₂[COD]BF₄:
MS(ESI/pos. in CH₂Cl₂): m/z = 795 [M⁺-BF₄-COD].

Rh[(*R*)**Ic**]₂[COD]BF₄:
MS(ESI/pos. in CH₂Cl₂): m/z = 897 [M⁺-BF₄-COD-2H].

Rh[(*R*)**IIa**][(*R*)**Ic**][COD]BF₄:
MS(ESI/pos. in CH₂Cl₂): m/z = 845 [M⁺-BF₄-COD-2H].

## Patentansprüche

1. Chiraler Übergangsmetall-Katalysator, **dadurch gekennzeichnet, dass** am Metall, ausgewählt aus Rh, Ir, Ru, Ni, Pd oder Pt, mindestens zwei strukturell unterschiedliche Mono-Phosphor-Liganden gebunden sind, wobei mindestens ein Mono-Phosphor-Ligand chiral ist, der ausgewählt ist aus Mono-P-Verbindungen vom Typ B, C oder D, 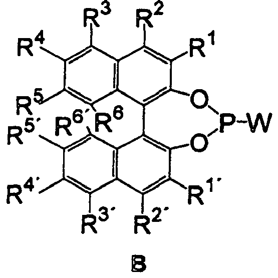 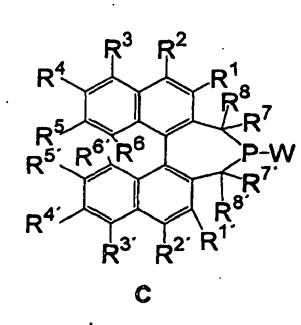 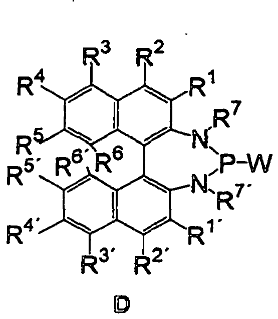 wobei W für Kohlenstoff (C), Stickstoff (N), Sauerstoff (O), Schwefel (S) oder Halogen (F, Cl, Br, I) steht und an W entsprechend seiner Anzahl von freien Valenzen weitere Atome oder Gruppen von Atomen gebunden sind,
und wobei die Reste R¹, R², R³, R⁴, R⁵, R⁶, R⁷, R⁸, R^{1'}, R^{2'}, R^{3'}, R^{4'}, R^{5'}, R^{6'}, R^{7'} und R^{8'} unabhängig voneinander aus der Reihe Wasserstoff, Halogen, gesättigte und ungesättigte lineare und verzweigte C₁-C₅₀ Alkyl-, C₆-C₅₀ Aryl-, C₁-C₅₀ Heteroaryl-, Alkinyl-, Silyl-, Nitro-, Nitril-, Ester-, Carboxyl-, Carbonyl-, Amid-, Amin-, Hydroxy-, Alkoxy-, Sulfid- und Selenidgruppen ausgewählt sind,
wobei R¹, R², R³, R⁴, R⁵, R⁶, R⁷, R⁸, R^{1'} R^{2'}, R^{3'}, R^{4'}, R^{5'}, R^{6'}, R^{7'} und R^{8'} ihrerseits weitere Substituenten tragen oder funktionalisiert sein können.

2. Chiraler Übergangsmetall-Katalysator nach Anspruch 1, wobei mindestens zwei Mono-Phosphor-Liganden chiral sind.

3. Chiraler Übergangsmetall-Katalysator nach einem der Ansprüche 1 oder 2, wobei die Mono-Phosphor-Liganden Phosphane, Phosphite, Phosphonite, Phosphinite, Phosphorigsäuretrisamide, Phosphorigsäuremonoesterdiamide, Phosphorigsäurediesteramide, Phosphonigsäurediamide, Phosphinigsäureamide, Phosphonigsäuremonoesteramide, Phosphorigsäurehalogenide, Phosphorigsäurediamidhalogenide, Thiophosphite, Thiophosphorigsäuretriester, Thiophosphorigsäuremonoesterdiamide oder Thiophosphorigsäurediesteramide sind.

4. Chiraler Übergangsmetall-Katalysator nach einem der Ansprüche 1 bis 3, wobei mindestens ein achiraler Ligand eine Mono-P-Verbindung vom Typ H-T 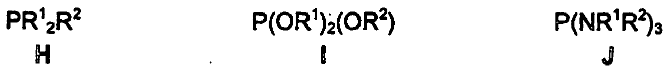 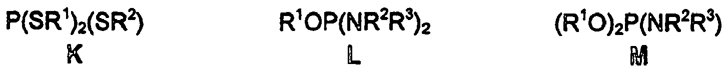 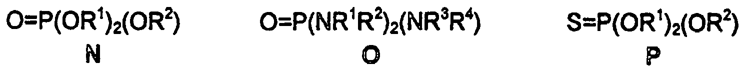 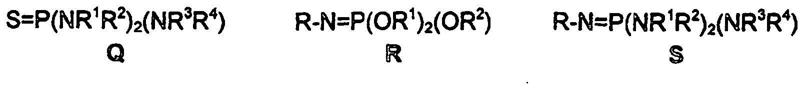 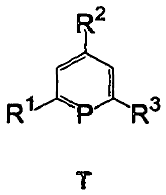 ist,
wobei die Reste R¹, R², R³ und R⁴ unabhängig voneinander aus der Reihe Wasserstoff, Halogen, gesättigte und ungesättigte lineare und verzweigte C₁-C₅₀ Alkyl-, C₆-C₅₀ Aryl-, C₁-C₅₀ Heteroaryl-, Alkinyl-, Silyl-, Nitro-, Nitril-, Ester-, Carboxyl-, Carbonyl-, Amid- und Selenidgruppen ausgewählt sind,
wobei R¹, R², R³ und R⁴ ihrerseits weitere Substituenten tragen, funktionalisiert oder verbrückt sein können.

5. Chiraler Übergangsmetall-Katalysator nach einem der Ansprüche 1 bis 4, wobei als chirale Mono-Phosphor-Liganden mindestens zwei Liganden des Typs 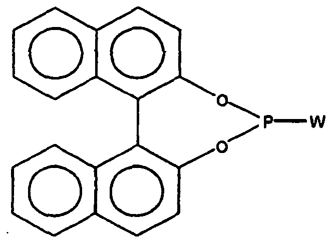 verwendet werden, wobei W unabhängig voneinander CH₃, C(CH₃)₃, *c*-C₆H₁₁ oder OCH₃ ist.

6. Verfahren zur katalytischen Herstellung von chiralen organischen Verbindungen, bei dem prochirale organische Verbindungen in Gegenwart eines chiralen Übergangsmetallkatalysators nach einem der Ansprüche 1 bis 5 hydriert werden.

## Claims

1. A chiral transition metal catalyst, **characterized in that** at least two structurally different monophosphorus ligands are bonded to the metal selected from Rh, Ir, Ru, Ni, Pd or Pt, at least one monophosphorus ligand being chiral which is selected from mono-P-compounds of the B, C or D type: 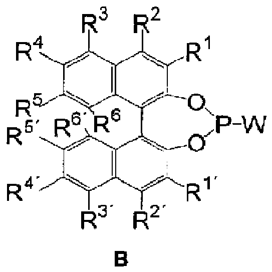 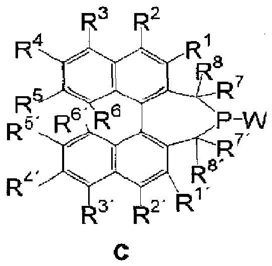 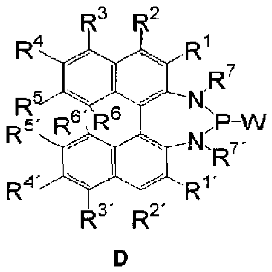 where W is carbon (C), nitrogen (N), oxygen (O), sulfur (S) or halogen (F, Cl, Br, I),
and further atoms or groups of atoms are bonded to W according to its number of free valences,
and where the radicals R¹, R², R³, R⁴, R⁵, R⁶, R⁷, R⁸, R^{1'}, R^{2'}, R^{3'}, R^{4'}, R^{5'}, R^{6'}, R^{7'} and R^{8'} are each independently selected from the group of hydrogen, halogen, saturated and unsaturated, linear and branched C₁-C₅₀ alkyl, C₆-C₅₀ aryl, C₁-C₅₀ heteroaryl, alkynyl silyl, nitro, nitrile, ester, carboxyl, carbonyl, amide, amine, hydroxyl, alkoxy, sulfide and selenide groups,
where R¹, R², R³, R⁴, R⁵, R⁶, R⁷, R⁸, R^{1'}, R^{2'}, R^{3'}, R^{4'}, R^{5'}, R^{6'}, R^{7'} and R^{8'} in turn bear further substituents or may be functionalized.

2. A chiral transition metal catalyst as claimed in claim 1, wherein at least two monophosphorus ligands are chiral.

3. A chiral transition metal catalyst as claimed in any of claims 1 or 2, wherein the monophosphorus ligands are phosphines, phosphites, phosphonites, phosphinites, phosphorous triamides, phosphorous monoester diamides, phosphorous diester amides, phosphonous diamides, phosphinous amides, phosphonous monoester amides, phosphorous halides, phosphorous diamide halides, thiophosphites, thiophosphorous triesters, thiophosphorous monoester diamides or thiophosphorous diesteramides.

4. A catalyst as claimed in any of claims 1 to 3, wherein at least one achiral ligand is a monophosphorus compound of the H-T type 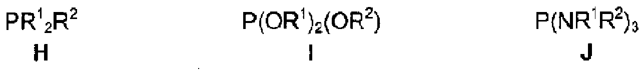 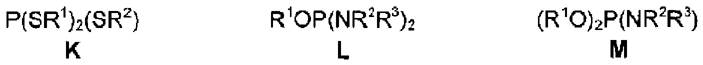 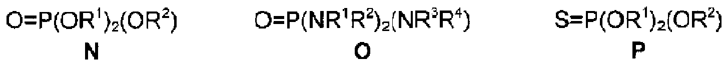 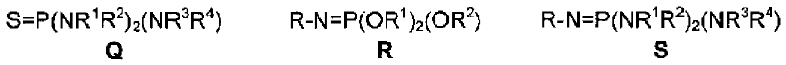 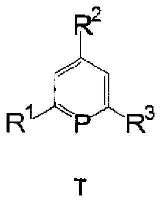 wherein the radicals R¹, R², R³ and R⁴ are each independently selected from the group of hydrogen, halogen, saturated and unsaturated, linear and branched C₁-C₅₀ alkyl, C₆-C₅₀ aryl, C₁-C₅₀ heteroaryl, alkynyl, silyl, nitro, nitrile, ester, carboxyl, carbonyl, amide and selenide groups,
where R¹, R², R³ and R⁴ in turn bear further substituents and may be functionalized or bridged.

5. A catalyst as claimed in any of claims 1 to 4, wherein the chiral monophosphorus ligands used are at least two ligands of the type 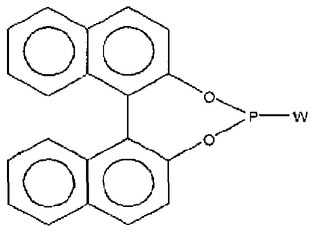 where W is each independently CH₃, C(CH₃)₃, c-C₆H₁₁ or OCH₃.

6. A process for catalytically preparing chiral organic compounds, **characterized in that** prochiral organic compounds are hydrogenated in the presence of a chiral transition metal catalyst as claimed in any of claims 1 to 5.

## Revendications

1. Catalyseur chiral de métal de transition, **caractérisé en ce qu'**au moins deux ligands monophosphorés structuralement différents sont liés sur le métal, choisi parmi Rh, Ir, Ru, Ni, Pd ou Pt, au moins un ligand monophosphoré étant chiral, qui est choisi parmi les composés mono-P du type B, C ou D, 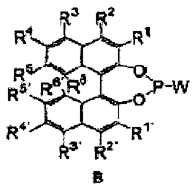 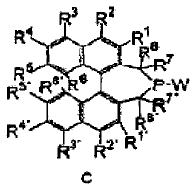 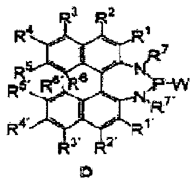 W représentant carbone (C), azote (N), oxygène (O), soufre (S) ou halogène (F, Cl, Br, I) et d'autres atomes ou groupes d'atomes étant liés sur W en fonction de son nombre de valences libres,
les radicaux R¹, R², R³, R⁴, R⁵, R⁶, R⁷, R⁸, R^{1'}, R^{2'}, R^{3'}, R^{4'}, R^{5'}, R^{6'}, R^{7'} et R^{8'} étant choisis, indépendamment les uns des autres, dans la série hydrogène, halogène, les groupes C₁-C₅₀-alkyle saturés et insaturés, linéaires et ramifiés, C₆-C₅₀-aryle, C₁-C₅₀-hétéroaryle, alcynyle, silyle, nitro, nitrile, ester, carboxyle, carbonyle, amide, amine, hydroxy, alcoxy, sulfure et séléniure, R¹, R², R³, R⁴, R⁵, R⁶, R⁷, R⁸, R^{1'}, R^{2'}, R^{3'}, R^{4'}, R^{5'}, R^{6'}, R^{7'} et R^{8'} pouvant à leur tour porter d'autres substituants ou pouvant être à leur tour fonctionnalisés.

2. Catalyseur chiral de métal de transition selon la revendication 1, au moins deux ligands monophosphorés étant chiraux.

3. Catalyseur chiral de métal de transition selon l'une quelconque des revendications 1 ou 2, les ligands monophosphorés étant des phosphanes, des phosphites, des phosphonites, des phosphinites, des triamides de l'acide phosphoreux, des monoester-diamides de l'acide phosphoreux, des diester-amides de l'acide phosphoreux, des diamides de l'acide phosphoneux, des amides de l'acide phosphinique, des monoester-amides de l'acide phosphoneux, des halogénures de l'acide phosphoreux, des diamide-halogénures de l'acide phosphoreux, des thiophosphites, des triesters de l'acide thiophosphoreux, des monoester-diamides de l'acide thiophosphoreux ou des diester-amides de l'acide thiophosphoreux.

4. Catalyseur chiral de métal de transition selon l'une quelconque des revendications 1 à 3, au moins un ligand achiral étant un composé mono-P de type H-T 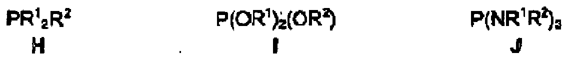 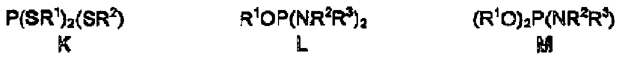 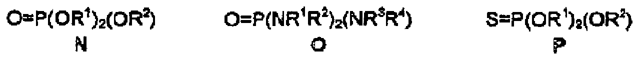 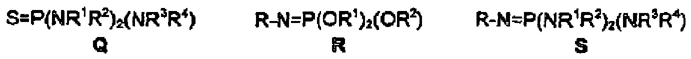 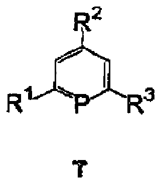 les radicaux R¹, R², R³ et R⁴ étant choisis, indépendamment les uns des autres, dans la série hydrogène, halogène, les groupes C₁-C₅₀-alkyle saturés et insaturés, linéaires et ramifiés, C₆-C₅₀-aryle, C₁-C₅₀-hétéroaryle, alcynyle, silyle, nitro, nitrile, ester, carboxyle, carbonyle, amide et séléniure,
R¹, R², R³ et R⁴ pouvant à leur tour porter d'autres substituants, être fonctionnalisés ou être pontés.

5. Catalyseur chiral de métal de transition selon l'une quelconque des revendications 1 à 4, utilisant, comme ligands monophosphorés chiraux, au moins deux ligands du type 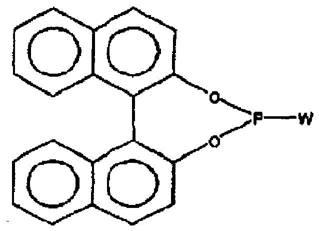 W représentant, indépendamment les uns des autres, CH₃, C(CH₃)₃, c-C₆H₁₁ ou OCH₃.

6. Procédé pour la préparation catalytique de composés organiques chiraux, dans lequel des composés organiques prochiraux sont hydrogénés en présence d'un catalyseur chiral de métal de transition selon l'une quelconque des revendications 1 à 5.
